# EUROPÄISCHE PATENTSCHRIFT

(11) **EP 0 732 112 B1**
(45) Veröffentlichungstag und Bekanntmachung des Hinweises auf die Patenterteilung: **23.02.2000**
(21) Anmeldenummer: 96103911.2
(22) Anmeldetag: 13.03.1996
(51) Int. Cl.: A61M 5/142

(54) **Implantierbare Infusionspumpe**
Implantable infusion pump
Pompe de perfusion implantable

(30) Priorität: 17.03.1995 DE 19509632
(43) Veröffentlichungstag der Anmeldung: 18.09.1996
(73) Patentinhaber: Fresenius AG, 61350 Bad Homburg v.d.H (DE)
(72) Erfinder: Steinbach, Bernd, Dr., 61169 Friedberg (DE)
(74) Vertreter: Luderschmidt, Schüler & Partner GbR

(56) Entgegenhaltungen:
- WO-A-92/09316

## Beschreibung

Gegenstand der vorliegenden Erfindung ist eine implantierbare Infusionspumpe zur dosierten Abgabe von Medikamenten in den menschlichen Körper nach dem Oberbegriff des Patentanspruches 1.

Implantierbare Infusionspumpen sind bereits bekannt. Aus der DE 39 15 251 A1 ist eine implantierbare Infusionspumpe bekannt, die eine Pumpenkammer aufweist, die durch ein schalenförmiges Kammerunterteil und ein damit verbundenes Kammeroberteil gebildet ist. Die Pumpenkammer ist durch eine flexible Membran in zwei Teilkammern getrennt. Eine erste Teilkammer ist vom Kammeroberteil und der Membran begrenzt und als Medikamentenspeicher ausgebildet. Das Kammeroberteil enthält eine Nachfüllöffnung, die mit einem durchstechbaren Septum abgedichtet ist. Das Septum ist zwischen einem mit dem Kammeroberteil verbundenden Septenhalter und dem Kammeroberteil verklemmt. Der Medikamentenspeicher ist über eine Auslaßöffnung und gegebenenfalls eine Auslaßreduziereinrichtung mit einem Auslaßkatheter verbunden. Eine zweite Teilkammer wird vom Kammerunterteil und der Membran begrenzt und ist als Druckkammer zur Aufnahme eines sich bei Körpertemperatur ausdehnenden Treibmittels vorgesehen.

Infusionspumpen mit einem ähnlichen Aufbau sind weiter aus der DE 26 04 113 A1, der DE 21 24 062 B2 sowie aus der DE 40 38 049 A1 bekannt. Eine weitere Infusionspumpe wird in der DE 44 32 991 A1 beschrieben, auf die hierin zu Offenbarungszwecken ausdrücklich Bezug genommen wird.

Die Pumpenbauteile bestehen bei diesen Infusionspumpen aus körperverträglichen Metall-Legierungen oder Kunststoffen, die durch Schweißverbindungen oder Rastverbindungen miteinander verbunden sind.

Implantierbare Infusionspumpen werden in einer subkutanen Tasche im Bereich des Abdomens des Patienten angeordnet, wobei die mit dem Septum verschlossene Nachfüllöffnung unter der Haut des Patienten tastbar ist. Der Medikamentenspeicher wird dadurch gefüllt, daß man eine Spritze mit einer entsprechenden Kanüle durch die Haut des Patienten und durch das Septum sticht. Das Medikament fließt aufgrund des Spritzendrucks durch die Kanüle in den Medikamentenspeicher.

Implantierbare Infusionspumpen werden zur kontinuierlichen Medikation (gleichbleibende Dosis) über längere Zeiträume bei Patienten eingesetzt, die sonst nur durch Injizieren der Medikamente, z.B. von Morphinen, Heparinen und dergleichen, mehrmals täglich behandelt werden können. Die Pumpen haben gegenüber der üblichen Injektion den Vorteil, daß die verabreichte Dosis nicht mehr so weit überdosiert werden muß, daß trotz des Abbaus des Medikamentes bis zum nächsten Verabreichungzeitpunkt eine gewisse Mindestdosis nicht unterschritten wird, sondern eine gleichmäßige und insgesamt wesentlich verringerte Zufuhr des Medikamentes verwirklicht werden kann. An solche Infusionspumpen müssen, insbesondere bei Gabe von Schmerzmitteln, hohe Sicherheitsanforderungen gestellt werden. Eine eventuelle Über- oder Unterdosierung muß vermieden werden. Eine Überdosierung kann abhängig vom verabreichten Medikament zu einem hohen Gesundheitsrisiko führen, insbesondere bei Schmerzmitteln sogar tödlich wirken.

Die Wirkungsweise einer implantierbaren Infusionspumpe ist im wesentlichen der Art, daß das in der Pumpe enthaltene Treibmittel, dessen Siedepunkt unterhalb der Körpertemperatur liegt, nach Implantation der Pumpe im Körper des Patienten teilweise verdampft und über die Trennwand, wie z.B. eine flexible Membran oder einen Faltenbalg, Druck auf das im Medikamentenspeicher befindliche Medikament ausübt, worauf das Medikament über ein Reduziersystem und einen Katheter zum Zielorgan fließt. Dabei verkleinert sich der Medikamentenraum unter Vergrößerung des Treibgasraumes. Die Volumenvergrößerung des Treibgasraumes wird von einer weiteren Verdampfung des Treibmittels aufgefangen und geschieht bei konstanter Temperatur so lange isobar, als das Treibmittel als Zweiphasensystem vorliegt und keine Fremdgase im Treibmittelraum existieren. Die isobare Druckentwicklung ist für die Systeme zur Beibehaltung einer konstanten Förderrate notwendig.

Die bisher in den implantierbaren Infusionspumpen verwendeten Treibmittel entsprechen nicht den an sie gestellten Anforderungen. Sie werfen zahlreiche Probleme auf.

In der aus der DE 40 38 049 A1 bekannten Infusionspumpe wird als Treibmittel 1,2-Dichlortetrafluorethan verwendet. Dieses Treibmittel entwickelt bei Körpertemperatur (etwa 37°C) einen Druck von über 2,5 bar über Atmosphärendruck. Dieser Druck muß bei Nachfüllung des Medikamentes überwunden werden, was an den mit der Nachfüllung betrauten Arzt hohe Anforderungen stellt und den Patienten belastet. Außerdem ist dieses Treibmittel sicherheitstechnisch bedenklich, da die Wände des Implantates höher belastet werden. Eine geringere sicherheitstechnische Belastung der Wände des Implantates wird bei Verwendung von Frigen 11 erreicht. Frigen 11 ist jedoch, wie das 1,2-Dichlortetrafluorethan, ein Fluorchlorkohlenwasserstoff (FCKW), auf deren Verwendung aufgrund ihrer nachteiligen Auswirkungen auf die Umwelt (u.a. Ozonabbau) verzichtet werden muß. Frigen 11 entwickelt bei Körpertemperatur einen Druck von lediglich etwa 0,6 bar über Atmosphärendruck und wäre aus sicherheitstechnischen Gründen einer Anwendung von 1,2-Dichlortetrafluorethan vorzuziehen. Bei dem gemäß der DE 40 38 049 A1 als Treibmittel verwendeten 1,2-Dichlortetrafluorethan ist die Förderrate der Infusionspumpe weniger von Temperaturänderungen (z.B. bei Fieber des Patienten) und Änderungen des Gegendruckes am Katheterende (z.B. Blutdruckänderungen, Luftdruckänderungen etwa bei Flugreisen oder Aufenthalt im Gebirge) abhängig als bei Verwendung von z.B. Frigen 11. Aus den genannten sicherheitstechnischen Gründen wäre dennoch Frigen 11 dem 1,2-Dichlortetrafluorethan der Vorzug zu geben.

Weitere Probleme ergeben sich bei bisher angewandten Treibmitteln aus ihrer relativen Unverträglichkeit gegenüber den sie umgebenden Materialien, wie Metallen und Kunststoffen, insbesondere gegenüber Kunststoffen.

Aufgabe der vorliegenden Erfindung ist daher die Bereitstellung einer implantierbaren Infusionspumpe, die kein FCKW als Treibmittel enthält, sicherheitstechnisch unproblematisch ist, bei der bei Temperaturerhöhungen infolge Fieber des Patienten (42°C) ein Maximaldruck von 2 bar nicht überschritten wird, die Förderrate vom Medikamentenvolumen unabhängig ist und ein relativ geringer Temperaturgradient vorliegt, die sicher und einfach zu handhaben ist, medikamentenverträglich ist, aus körperverträglichem Material und miteinander verträglichen Materialien besteht und einfach und kostengünstig herstellbar ist.

Diese Aufgabe wird bei einer implantierbaren Infusionspumpe der eingangs genannten Art dadurch gelöst, daß erfindungsgemäß das Treibmittel Hexafluorbutan ist.

Vorzugsweise ist das Treibmittel 1,1,1,4,4,4-Hexafluorbutan.

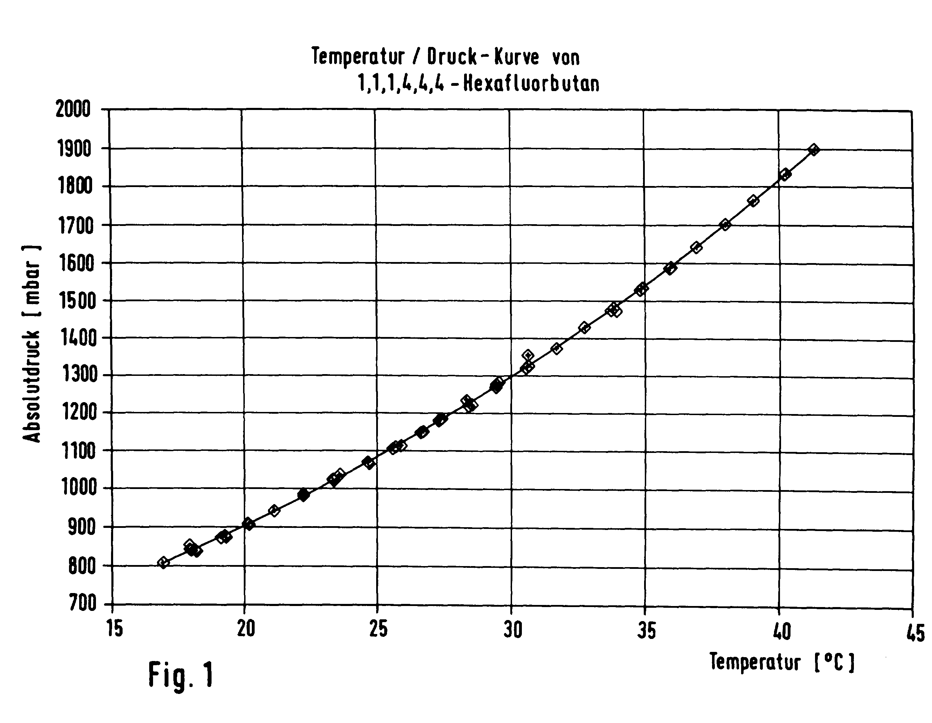

Das erfindungsgemäß verwendete Treibmittel besitzt einen Siedepunkt von 23°C. Es ist ungiftig und gegenüber den in der Infusionspumpe angewandten Materialien, insbesondere gegenüber Kunststoffen inert. Hinsichtlich seines Druck-Temperatur-Verhaltens liegt das erfindungsgemäß verwendete Treibgas ziemlich nahe an Frigen 11. Bei Körpertemperatur (etwa 37°C) entwickelt das Treibmittel einen Druck von etwa 670 mbar über Atmosphärendruck. Die beiliegende Figur 1 stellt eine Temperatur/Druck-Kurve des erfindungsgemäß bevorzugt verwendeTreibmittels dar. Aus ihr ergibt sich für das erfindungsgemäß verwendete Treibmittel ein relativ geringer Temperaturgradient. Die Druckerhöhung pro Temperaturänderung von 1°C beträgt maximal 70 mbar und liegt insbesondere im Bereich von 55-67 mbar. Im Hinblick auf diese Eigenschaften ist das erfindungsgemäß verwendete Treibmittel auch sicherheitstechnisch als unbedenklich anzusehen. Es besteht somit keine starke Belastung für das Gehäuse der erfindungsgemäßen Infusionspumpe. Ferner erfordert das Nachfüllen des Medikamentes keine hohen Spritzenkräfte durch den damit betrauten Arzt und werden die Belastungen für den Patienten verringert.

Die Befüllung der Druckkammer mit dem Treibmittel kann nach Entfernung der vorhandenen Luft aus der Druckkammer (z.B. durch Absaugen) in jeder geeigneten Weise erfolgen. Z.B. kann durch die Dichtfläche eine Mikrokapillare eingeführt werden, durch welche das Treibmittel in den Druckraum eingefüllt wird. Nach Befüllen mit dem Treibmittel kann die Kapillare vergossen oder herausgezogen werden. Die eingefüllte Treibmittelmenge ist gering und kann beispielsweise 2 ml betragen, während das Innenvolumen des Druckraumes z.B. etwa 40 ml betragen kann.

Die Infusionspumpen können in jeder geeigneten Form gestaltet und aus jedem geeigneten körperverträglichen Material, wie Metall oder Kunststoff hergestellt sein. Vorzugsweise werden Kunststoffe verwendet, wobei Hartkunststoffe, wie Polysulfone (einschließlich Polyethersulfone), Polyamide und Polycarbonate, insbesondere Polysulfone, bevorzugt sind. Die Verwendung von Infusionspumpen aus Kunststoffen bringt erhebliche Vorteile. Abgesehen von dem geringeren Gewicht gegenüber Infusionspumpen aus Metall wird mit Infusionspumpen aus Kunststoffen die Voraussetzung für die gleichzeitige Anwendung bestimmter therapeutischer oder diagnostischer Verfahren, wie Thermographie oder NMR geschaffen.

Insbesondere sind die Infusionspumpen so ausgebildet, wie sie in der DE 44 32 991 A1 und/oder der gleichzeitig mit der vorliegenden Patentanmeldung eingereichten Patentanmeldung mit dem Aktenzeichen P 195 09 634.7-35 mit dem Titel "Implantierbare Infusionspumpe" (unser Zeichen FR2665) beschrieben sind.

Die erfindungsgemäße Infusionspumpe ist zur kontinuierlichen dosierten Abgabe von Medikamenten, wie z.B. Heparin, künstlichem Pankreasinsulin, Chemotherapeutika, Schmerzmittel, wie Morphium, Muskelrelaxantien, wie Baclofen und dergleichen geeignet.

Die nachfolgenden Beispiele dienen der weiteren Erläuterung der vorliegenden Erfindung.

### Beispiel 1

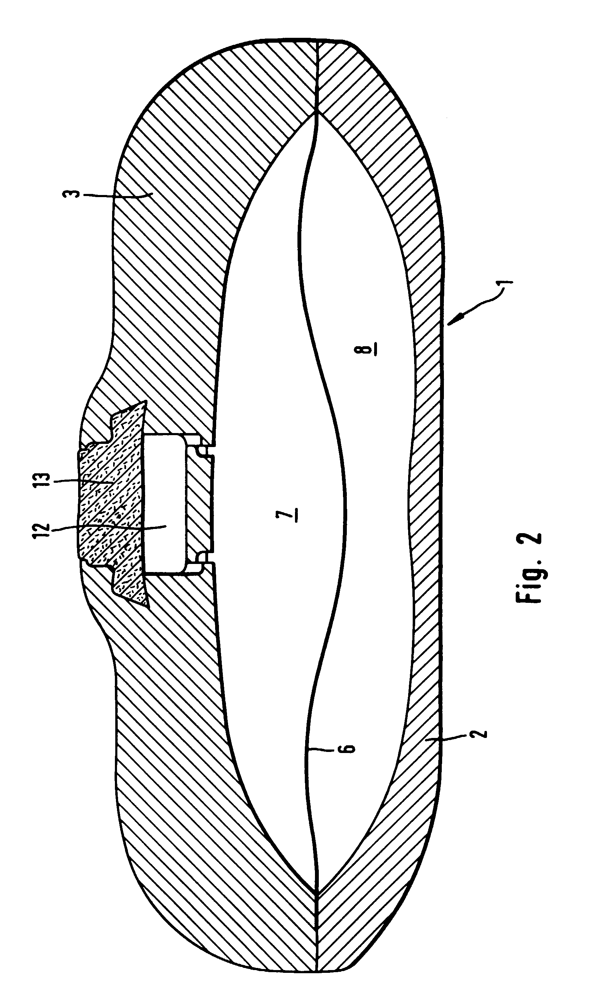

Die Figur 2 stellt einen Vertikalschnit durch eine schematisch veranschaulichte erfindungsgemäße implantierbare Infusionspumpe dar. Die Infusionspumpe 1 ist ein scheibenförmiger rotationssymmetrischer Körper aus Hartkunststoff mit einer Pumpenkammer, die durch ein Kammerunterteil 2 und ein Kammeroberteil 3 gebildet ist und durch die flexible gasundurchlässige Trennwand 6 in zwei Teilkammern 7 und 8 getrennt ist. Die erste Teilkammer 7 dient als Medikamentenspeicher und die zweite Teilkammer 8 dient als Druckkammer zur Aufnahme des als Treibmittel eingesetzten 1,1,1,4,4,4-Hexafluorbutan. Die Nachfüllöffnung 12 ist durch ein durchstechbares Zentralseptum 13 dicht abgedeckt und weist unter dem Zentralseptum 13 einen Nachfüllraum mit einer festen Platte als Nadelstop und Durchtrittsöffnungen zum Medikamentenspeicher 7 auf.

Die flexible gasundurchlässige Trennwand 6 ist eine üblicherweise verwendete flexible Membran.

Gemäß einer weiteren Ausführungsform ist die flexible gasundurchlässige Trennwand 6 der Kontur der Innenform des Kammeroberteils 3 (und entsprechend des Kammerunterteils 2) folgend gewölbt, was durch Tiefziehen erreicht wurde. Diese Trennwand ist spannungsfrei. Sie besteht aus einem Foliensystem, das, vom Medikamentenspeicher 7 aus gesehen, folgendermaßen zusammengesetzt ist:
a) Folie aus Polyethylenterphthalat, Dicke 100 µm
b) Folie aus Polychlortrifluorethylen, Dicke 127 µm
c) Aluminiumverbundfolie aus
   i) Polyethylenterphthalatfolie, Dicke 12 µm
   ii) Aluminiumfolie, Dicke 12 µm
   iii) Polyethylenfolie, Dicke 70 µm.

Die Folien der Aluminiumverbundfolie sind mittels eines Polyurethanklebers miteinander verklebt. Die Folien a) und b) sind lediglich im Randbereich entlang des gesamten Umfangs miteinander und der Aluminiumverbundfolie verbunden. Die Trennwand 6 ist mit ihrem äußeren Randbereich entlang ihres Umfangs zwischen die Randbereiche des Kammeroberteils 3 und des Kammerunterteils 2 eingeklemmt bzw. eingepreßt.

Das als Treibmittel verwendete 1,1,1,4,4,4-Hexafluorbutan wurde derart in die Druckkammer gebracht, daß man durch die Dichtfläche im Randbereich eine Mikrokapillare einführte, die Luft aus der Druckkammer entfernte und durch die Mikrokapillare das Treibmittel in den Druckraum einfüllte. Die eingefüllte Menge betrug etwa 2 ml.

Das Innenvolumen des Druckraumes 8 beträgt etwa 40 ml, der Medikamentenspeicher 7 kann etwa 30 ml der medizinischen Lösung aufnehmen.

### Beispiel 2

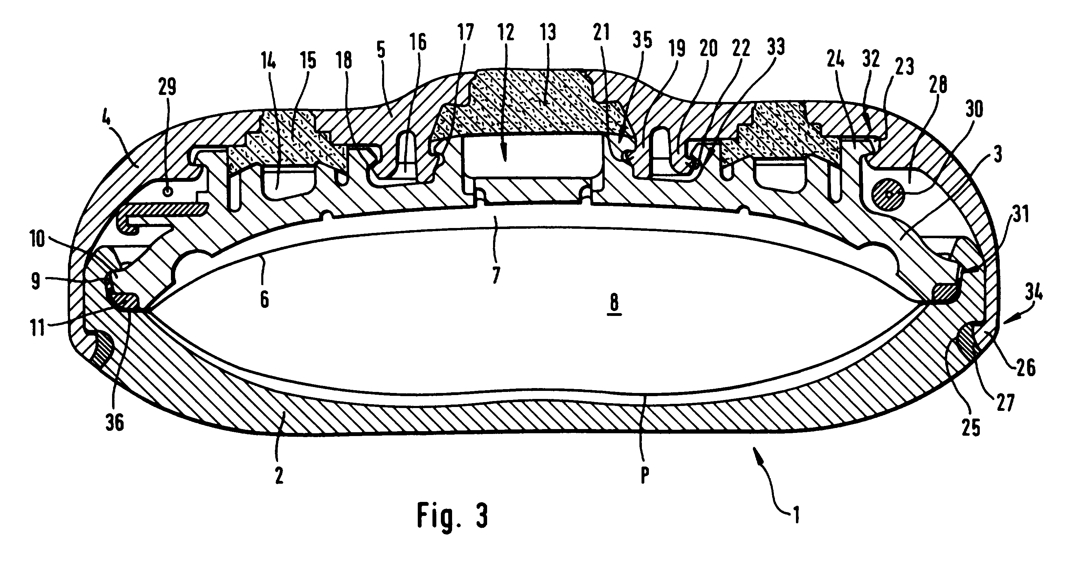

In Figur 3 ist ein Vertikalschnitt durch eine speziell gestaltete Infusionspumpe zur dosierten Abgabe von Medikamenten in den menschlichen Körper dargestellt.

Die Infusionspumpe 1 ist ein scheibenförmiger rotationssymmetrischer Körper aus Polysulfon, die ein Gehäuse aufweist, das aus einem schalenförmigen Kammerunterteil 2, einem schalenförmig mit entgegengesetzter Wölbung ausgeführtem Kammeroberteil 3, einem Überwurfteil 4 und einem Septenhalter 5 zusammengesetzt ist. Der Innenraum ist durch eine gewölbte Trennwand 6 und eine ebenfalls gewölbte Aluminiumverbundfolie P in eine erste Teilkammer, die als Medikamentenspeicher 7 dient, und eine zweite Teilkammer, die als Druckkammer 8 zur Aufnahme des als Treibmittel verwendeten 1,1,1,4,4,4-Hexafluorbutan dient, geteilt. Die Druckkammer 8 wird durch Randverschweißung oder -verklebung der Trennwand 6 und der Aluminiumverbundfolie P gebildet und ist pillenförmig gestaltet.

Am oberen inneren Randbereich des Kammerunterteils 2 ist eine Ringnut eingeformt, in die in einer Rastverbindung ein umlaufender zugeordneter Rand 10 des Kammeroberteils 3 zusammen mit einem O-Ring 11 und einem Rand 36, der aus der Trennwand 6 und der Aluminiumverbundfolie P gebildet wird, eingerastet und dicht gehalten ist.

Eine Nachfüllöffnung 12 ist durch ein scheibenförmiges und durchstechbares Zentralseptum 13 dicht abgedeckt, wobei die Nachfüllöffnung 12 einen Nachfüllraum unter dem Zentralseptum 13 mit einer festen Platte als Nadelstopp und Durchtrittsöffnungen zum Medikamentenspeicher 7 aufweist.

Weiter ist an der Oberseite des Kammeroberteils 3 eine konzentrische Ringkammer 14 eingeformt, die von einem Ringseptum 15 dicht abgedeckt ist.

Zwischen der Ringkammer 14 und der Nachfüllöffnung 12 ist eine konzentrische Ringnut 16 eingeformt, an deren innerer und äußerer Seitenwand jeweils eine umlaufene Rastnut 17, 18 angebracht ist. Der ringförmige Septenhalter 5 übergreift mit seinem inneren Rand den Rand des scheibenförmigen Zentralseptums 13 und mit seinem äußeren Rand den ringinneren Rand des Ringseptums 15. Zudem greift der Septenhalter 5 mit nebeneinander liegenden Ringstegen 19, 20 in die Ringnut 16 ein, wobei die Rastnasen 21, 22 auf den Ringstegen 19, 20 in die Rastnuten 17, 18 eingreifen.

Der ringäußere Rand des Ringseptums 15 ist in einer stufenförmigen Anordnung von einem entsprechend geformten Randbereich des Überwurfteils 4 überdeckt und gegen das Kammeroberteil 3 dicht verklemmt. Dazu ist in diesem Randbereich des Überwurfteils 4 eine Rastverbindung zwischem dem Überwurfteil 4 und dem Kammeroberteil 3 vorgesehen, die aus einer umlaufenden Rastnut 23 am Überwurfteil 4 und einer umlaufenden Rastnase 24 am Kammeroberteil 3 besteht.

Am seitlichen äußeren Randbereich des Kammerunterteils 2 ist eine weitere Ringnut 25 eingeformt, in die eine umlaufende Rastnase 26 zusammen mit einem eingelegten O-Ring 27 eingreift. Die Rastnase 26 stellt den unteren Rand des Überwurfteils 4 dar, wobei dieses den seitlichen Bereich des Kammeroberteils 3 und den äußeren seitlichen Randbereich des Kammerunterteils 2 glockenförmig übergreift. Das Überwurfteil 4 stützt sich (bei großer Druckbelastung) am Kammeroberteil 3 über die Rastnasenanordnung 24 und über das Ringseptum 15 zum Kammerunterteil 2 hin ab.

Zwischen dem äußeren Wandbereich des Kammeroberteils 3 und einem inneren Wandbereich des Überwurfteils 4 ist ein Ringraum 28 zur Aufnahme einer Auslaßreduziereinrichtung 29 (hier schematisch als Schnitt durch eine Kapillare dargestellt) und eines Auslaßkatheters 30 gebildet.

Die Verbindung 31, gebildet aus der Ringnut 9, dem Rand 10 und dem O-Ring 11, die Verbindung 32, gebildet aus der Rastnut 23 und der Rastnase 24 sowie die Verbindung 33, bestehend aus der Rastnut 18 und den Rastnasen 22, stellen Hauptverbindungen dar, die bei normaler betriebsmäßiger Belastung die auftretenden Innendruckbelastungen im wesentlichen abstützen.

Die Verbindung 34, bestehend aus der Ringnut 25, der Rastnase 26 und dem O-Ring 27, sowie die Verbindung 35, bestehend aus der Rastnut 17 und den Rastnasen 21, sind Nebenverbindungen, die bei normaler betriebsmäßiger Belastung noch keine oder nur eine geringe Abstützfunktion haben. Erst bei einer Druckerhöhung über den normalen Betriebsdruck, insbesondere bei einem Versagen der Hauptverbindungen 31, 32, 33 übernehmen die Nebenverbindungen 34, 35 eine Abstützfunktion.

Die Hauptverbindungen 31, 32, 33 stehen dazu in dauerndem Eingriff und stellen die Integrität der implantierten Infusionspumpe unter Normalbedingungen sicher. Bei Erhöhung des Innendrucks entweder im Ringraum 28 oder im Medikamentenspeicher 7 kommen die Nebenverbindungen 34, 35 zunehmend in Eingriff. Dadurch werden die Spannungen von den Hauptverbindungen 31, 32, 33 abgeleitet und teilen sich auf Haupt- und Nebenverbindungen auf.

Sollte beispielsweise die Hauptverbindung 31 versagen, so wird die Verbindungsaufgabe von der Nebenverbindung 34 übernommen. Da gleichzeitig ein Spalt zwischen Kammeroberteil 3 und Kammerunterteil 2 entsteht, entweicht Medikament in den Ringraum 28 zwischen dem Überwurfteil 4 und dem Kammeroberteil 3, so daß auch dadurch Druck und damit die Belastung auf die Verbindung insgesamt abnimmt. Dadurch ist die Nebenverbindung 34 in der Lage, die Integrität der Infusionspumpe nach außen hin sicherzustellen. Wesentlich ist dabei, daß das Unterteil im oberen Stegbereich versagt, nicht aber im Unterbereich, was konstruktiv sichergestellt werden kann.

Bei einem Versagen der Hauptverbindung 32 zwischen dem Kammeroberteil 3 und dem Überwurfteil 4 wird die Verbindung ebenfalls durch die Nebenverbindung 34 übernommen.

Entsprechend wird bei einem Versagen oder einer Überlastung der Hauptverbindung 33 eine Übernahme oder Aufteilung auf die Nebenverbindung 35 übertragen.

In jedem Fall ergibt sich der Vorteil, daß bei einer Überlastung oder bei einem Versagen der Hauptverbindungen 31, 32, 33 Nebenverbindungen 34, 35 zur Verfügung stehen, die den sicheren Weiterbetrieb der Infusionspumpe 1 gewährleisten, wodurch die Sicherheit für einen Patienten erheblich verbessert ist.

Die in diesem Beispiel angewandte Trennwand 6 entspricht der in Beispiel 1 angewandten Trennwand 6 sowohl hinsichtlich Zusammensetzung als auch Gestalt. Die Aluminiumverbundfolie P ist durch Tiefziehen der Kontur der Innenwand des Kammerunterteils 2 folgend gewölbt und an den Rändern entlang des Umfanges mit der Trennwand 6 verbunden. Die Aluminiumverbundfolie P besitzt, von der Druckkammer 8 her gesehen, die folgende Zusammensetzung:
i) Polyethylenfolie, Dicke 70 µm
ii) Aluminiumfolie, Dicke 12 µm
iii) Polyethylentherphtalat-Folie, Dicke 12 µm.

Der so gebildete pillenförmige Druckraum 8 weist ein Innenvolumen von etwa 40 ml auf und enthält etwa 2 ml des Treibmittels. Der Medikamentenspeicher kann etwa 30 ml der medizinischen Lösung aufnehmen. Diese speziell gestaltete Infusionspumpe gestattet bis zu maximal 120 Füllungen mit der medizinischen Lösung und Förderraten pro Tag von z.B. 0,7, 1,0, 1,4 oder 2 ml der medizinischen Lösung.

## Patentansprüche

1. Implantierbare Infusionspumpe (1) zur dosierten Abgabe von Medikamenten in den menschlichen Körper, mit einer Pumpenkammer, die durch ein Kammerunterteil (2) und ein damit verbundenes Kammeroberteil (3) gebildet ist, wobei
a) die Pumpenkammer durch eine gasundurchlässige flexible Trennwand (6) in zwei Teilkammern (7, 8) getrennt ist,
b) die erste Teilkammer (7) vom Kammeroberteil (3) und der flexiblen Trennwand (6) begrenzt ist und als Speicher für medizinische Lösungen (7) ausgebildet ist, das Kammeroberteil (3) eine Nachfüllöffnung (12) aufweist, die durch wenigstens ein durchstechbares Septum (13) abgedichtet ist, und der Speicher (7) für medizinische Lösungen über eine Auslaßöffnung und gegebenenfalls eine Auslaßreduziereinrichtung (29) mit einem Auslaßkatheter (30) verbunden ist, und
c) die zweite Teilkammer vom Kammerunterteil (2) und der flexiblen Trennwand (6) begrenzt ist und als Druckkammer (8) zur Aufnahme eines Treibmittels aus halogeniertem Kohlenwasserstoff ausgebildet ist,
**dadurch gekennzeichnet**,
daß das Treibmittel Hexafluorbutan ist.

2. Infusionspumpe nach Patentanspruch 1, **dadurch gekennzeichnet**, daß das Treibmittel 1,1,1,4,4,4-Hexafluorbutan ist.

## Claims

1. Implantable fusion pumps (1) for dosing medication in the human body, with a pump chamber, consisting of a lower chamber section (2) and an attached top chamber section (3), whereby
a) the pump chambers are separated by a non gas permeable flexible dividing wall (6) into two part chambers (7, 8)
b) the first part chamber (7) is delimited by the top chamber section (3) and the flexible membrane (6) and forms a store for medical solutions (7), the top chamber section (3) has a refill port (12) which is sealed off at least by a septum (13) that can be pierced, and the store (7) for medical solutions is joined via a discharge port and if necessary a discharge reduction device (29) to a discharge catheter 930) and
c) the second part chamber is delimited by the lower chamber section (2) and the flexible membrane (6) and is intended as a pressure chamber (8) to take a propellant made of halogenated hydrocarbon
**characterised by the fact that**
the propellant is hexafluorobutane.

2. Infusion pump as per claim 1, **characterised by the fact that** the propellant is 1,1,1,4,4,4-hexafluorobutane.

## Revendications

1. Pompe de perfusion implantable (1) pour administration de doses de médicaments à l'organispme humain, comportant une chambre de pompe laquelle est formée par un partie inférieure de la chambre (2) et, reliée à celle-ci, une partie supérieure de la chambre (3),
a) la chambre de pompe est divisée en deux chambres partielles (7, 8) par une paroi de séparation souple imperméable aux gaz (6),
b) la première chambre partielle (7) est limitée par la partie supérieure de la chambre (3) et la paroi de séparation souple (6) et est formée en tant que réservoir de solutions médicinales (7), la partie supérieure de la chambre (3) présente un orifice de remplissage (12) lequel est étanché par un septum perforable (13) et le réservoir (7) de solutions médicinales est relié par l'intermédiaire d'un orifice de vidange et éventuellement d'un dispositif réducteur de vidange (29) à un cathéter de vidange (30), et
c) la seconde chambre partielle est limitée par la partie inférieure de la chambre (2) et la paroi de séparation souple (6) et est formée comme une chambre à pression (8) pour recevoir un agent propulseur lequel agent propulseur est un hydrocarbure halogéné,
**caractérisé en ce que**
l'agent propulseur est le 1,1,1,4,4,4-hexafluorobutane.

2. Pompe de perfusion selon la revendication 1, **caractérisée en ce que** l'agent propulseur est le 1,1,1,4,4,4-hexafluorobutane.
